# EUROPEAN PATENT APPLICATION

(11) **EP 2 127 651 A1**
(43) Date of publication of application: **02.12.2009**
(21) Application number: 08703452.6
(22) Date of filing: 18.01.2008
(51) Int. Cl.: A61K 31/445, A61K 47/02, A61K 47/08, A61K 47/16, A61K 47/30, A61K 47/36, A61K 47/42, A61P 25/28

(54) **STABILIZED MEDICINAL COMPOSITION CONTAINING DONEPEZIL, METHOD OF PRODUCING THE SAME AND METHOD FOR STABILIZATION**

(30) Priority: 19.01.2007 JP 2007010783
(71) Applicant: Eisai R&D Management Co., Ltd., Tokyo 112-8088 (JP)
(72) Inventor: HARADA, Tsutomu, Kakamigahara-shi Gifu 501-6195 (JP); WAKABAYASHI, Kenji, Kyoto-shi Kyoto 601-8025 (JP); MIYAMOTO, Yasunori, Kyoto-shi Kyoto 601-8025 (JP)
(74) Representative: HOFFMANN EITLE
(86) International application number: PCT/JP2008/050602
(87) International publication number: WO 2008/088039

(57) **Abstract**

An object of the present invention is to provide a novel pharmaceutical composition containing donepezil, which is already used as a remedy for the treatment of dementia (cognitive impairment), in order to increase options for administration methods and improve patient compliance. The present invention provides a pharmaceutical composition containing donepezil or a pharmaceutically acceptable salt thereof and a naturally-occurring polymer, the pharmaceutical composition further containing at least one of edetate, sulfite, dibutylhydroxytoluene and butylhydroxyanisole.

## Description

### TECHNICAL FIELD

The present invention relates to a pharmaceutical composition containing donepezil or a pharmaceutically acceptable salt thereof and a naturally-occurring polymer, and more particularly, to a stabilized pharmaceutical composition containing donepezil or a pharmaceutically acceptable salt thereof and a naturally-occurring polymer, a process of producing the same, and a method for stabilizing donepezil or a pharmaceutically acceptable salt thereof in the presence of a naturally-occurring polymer.

### BACKGROUND ART

In recent years, nursing care for persons suffering from senile dementia has become an important social issue, and development of drugs for the treatment thereof has become active. In particular, donepezil has been highly appreciated for its usefulness as a therapeutic drug for Alzheimer's dementia with acetyl cholinesterase inhibitory effect.

On the other hand, in the case of administering a drug to a patient, a dosage form such as a tablet, capsule, powder, granules, ointment, suppository or injectable preparation is suitably selected, and pharmaceutically acceptable additives such as vehicles and bases required for formulation are blended therein. At this time, even if the drug itself is stable with respect to heat or light, there are cases in which the drug may become unstable as a result of formulating with additives.

For example, donepezil is known to be unstable to light in a formulation with additives, and a method for stabilizing donepezil in said formulation by using an organic acid such as tosic acid or mesylic acid has been disclosed (see, for example, Patent Document 1).

In addition, it is known that, in compositions blended with the synthetic polymer - polyvinylpyrrolidone (hereinafter simply referred to as "PVP") - for the purpose of reducing the bitter taste of a drug, the blend of sodium sulfite reduces the amount of related substances that increase during storage of such compositions (see, for example, Patent Document 2).
Patent Document 1: Japanese Patent Application Laid-open No. H11-106353
Patent Document 2: Japanese Patent Application Laid-open No. 2000-136134

### DISCLOSURE OF INVENTION

### PROBLEM TO BE SOLVED BY THE INVENTION

An object of the present invention is to provide a novel pharmaceutical composition containing donepezil already used for the treatment of dementia (cognitive impairment), in order to increase options for administration methods and improve patient compliance.

### MEANS FOR SOLVING PROBLEM

Under the aforementioned circumstances, the present inventors examined various pharmaceutical compositions, and found that donepezil or a pharmaceutically acceptable salt thereof is decomposed in the case of being formulated with naturally-occurring polymers, and that related substances not observed in Patent Document 2 are formed. Therefore, the present inventors conducted extensive studies to enhance the stability of donepezil or a pharmaceutically acceptable salt thereof in a composition containing a naturally-occurring polymers, and found that this problem can be solved by the constitution described below, thereby leading to completion of the present invention.

Namely, in a first aspect thereof, the present invention provides a pharmaceutical composition containing donepezil or a pharmaceutically acceptable salt thereof and a naturally-occurring polymer, wherein the pharmaceutical composition further contains at least one of edetate, sulfite, dibutylhydroxytoluene and butylhydroxyanisole. In a preferable aspect of the pharmaceutical composition according to the present invention, the donepezil or pharmaceutically acceptable salt thereof is contained in a form such as a liquid, suspension, jelly, syrup, suppository, external preparation or injectable preparation.

In addition, in a second aspect thereof, the present invention provides a process of producing a pharmaceutical composition containing donepezil or a pharmaceutically acceptable salt thereof and a naturally-occurring polymer, wherein the process comprises mixing at least one of edetate, sulfite, dibutylhydroxytoluene and butylhydroxyanisole into said pharmaceutical composition.

Moreover, in a third aspect thereof, the present invention provides a method for stabilizing a pharmaceutical composition containing donepezil or a pharmaceutically acceptable salt thereof and a naturally-occurring polymer, wherein the method comprises adding at least one of edetate, sulfite, dibutylhydroxytoluene and butylhydroxyanisole to said pharmaceutical composition.

In addition, in a fourth aspect thereof, the present invention provides a stabilizer for a pharmaceutical composition containing donepezil or a pharmaceutically acceptable salt thereof and a naturally-occurring polymer, wherein the stabilizer contains at least one of edetate, sulfite, dibutylhydroxytoluene and butylhydroxyanisole.

### ADVANTAGEOUS EFFECT OF THE INVENTION

According to the present invention, a pharmaceutical composition containing donepezil or a pharmaceutically acceptable salt thereof and the naturally-occurring polymers is provided, wherein the pharmaceutical composition improves patient compliance, prevents accidental swallowing in patients with swallowing disorders and reduces management burden during the course of formulation. More specifically, even in compositions in which related substances form by decomposition of donepezil or a pharmaceutically acceptable salt thereof due to heat or change over time, because of coexisting naturally-occurring polymers, the blend of at least one of edetate, sulfite, dibutylhydroxytoluene and butylhydroxyanisole suppresses the increase of related substances derived from donepezil, thereby providing a pharmaceutical composition having improved storage stability.
In addition, according to the present invention, within a pharmaceutical composition containing the naturally-occurring polymers, a stabilizer for donepezil or a pharmaceutically acceptable salt thereof may be provided.
Moreover, according to the present invention, a simple process of producing a pharmaceutical composition containing donepezil or a pharmaceutically acceptable salt thereof may be provided, in order to stabilize the donepezil or pharmaceutically acceptable salt thereof.
Moreover, according to the present invention, a preparation containing donepezil or a pharmaceutically acceptable salt thereof can be provided irrespective of the type of additive, thereby increasing options for the administration methods.

### BEST MODE FOR CARRYING OUT THE INVENTION

Hereinafter, embodiments of the present invention are explained. The following embodiments are provided as exemplifications for explaining the present invention, and are not intended to limit the scope of the present invention. The present invention can be carried out in various forms without departing from the scope of the invention.

The present invention is based on the finding that related substances of donepezil or a pharmaceutically acceptable salt thereof increase when donepezil or a pharmaceutically acceptable salt thereof and a naturally-occurring polymer are coexisting in the form of a liquid, suspension, emulsion or semi-solid. More specifically, the amount of related substances derived from donepezil or a pharmaceutically acceptable salt thereof increase as a result of storing a composition in which both constituents are coexisting for a long period of time at room temperature or storing the composition under high-temperature conditions. Moreover, related substances found in the present invention were found to be different from related substances formed in compositions of Patent Document 2 in which the bitter taste of a drug has been reduced by incorporating PVP as a synthetic polymer. The present invention is based on the finding that the increase in the amount of related substances formed from donepezil or a pharmaceutically acceptable salt thereof can be inhibited by adding at least one of edetate, sulfite, dibutylhydroxytoluene and butylhydroxyanisole to the naturally-occurring polymers and donepezil or a pharmaceutically acceptable salt thereof.

The present invention provides a pharmaceutical composition containing donepezil or a pharmaceutically acceptable salt thereof having improved storage stability. Here, the donepezil used in the present invention is generally used for treatment of Alzheimer's dementia in the form of donepezil hydrochloride, and the structural formula thereof is as indicated below.

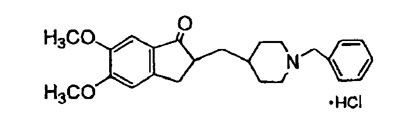

The term "pharmaceutically acceptable salt" used in present specification is not particularly limited, provided that it forms a salt with the donepezil used in the present invention and is pharmaceutically acceptable, examples of which may include inorganic acid salts, organic acid salts, and acidic amino acid salts. More specifically, preferable examples of inorganic acid salts may include hydrochlorides, hydrobromides, sulfates, nitrates and phosphates. Preferable examples of organic acid salts may include acetates, succinates, fumarates, maleates, tartrates, citrates, lactates, stearates, benzoates, methane sulfonates, ethane sulfonates, p-toluene sulfonates and benzene sulfonates. Preferable examples of acidic amino acid salts may include aspartates and glutamates. In the present invention, a more preferable pharmaceutically acceptable salt is a hydrochloride.

Examples of the naturally-occurring polymers used in the pharmaceutical composition according to the present invention may include pectin, carrageenan, agar, gelatin, gua gum, psyllium seed gum, starch, gellan gum, xanthan gum, locust bean gum, tara gum, tamarind gum, gum arabic, curdlan, galactomannan, glucomannan, nitrocellulose, methylcellulose, hydroxypropyl methylcellulose, alginic acid, sodium alginate, proteoglycan, glycoprotein, actin, tubulin, hemoglobin, insulin, fibrin, albumin, myosin, collagen, casein, pullulan, chitosan and mixtures thereof, with carrageenan, agar, xanthan gum, pectin or mixtures thereof being preferable, and pectin being more preferable.

The edetate (also referred to as ethylene diamine tetraacetic acid or EDTA) used in the pharmaceutical composition according to the present invention is not particularly limited, and examples of which may include sodium edetate and calcium disodium edetate, with calcium disodium edetate being preferable. Only one type of edetate may be added or a mixture of two or more types of edentate may be added. Moreover, one or more types of edetate, sulfite, dibutylhydroxytoluene and butylhydroxyanisole may be added separately or may be added as a mixture.

The sulfite used in the pharmaceutical composition according to the present invention is not particularly limited, and examples thereof may include sodium sulfite, sodium bisulfite, potassium sulfite, sodium pyrosulfite and potassium pyrosulfite, with sodium sulfite and sodium pyrosulfite being preferable. Only one type of sulfite may be added or a mixture of two or more types of sulfite may be added.

Although there are no particular limitations on the ratio of edetate, sulfite or dibutylhydroxytoluene in the pharmaceutical composition according to the present invention, the ratio is generally 0.0001 to 0.2 parts by weight and preferably 0.001 to 0.1 part by weight of edetate, sulfite or dibutylhydroxytoluene, and more preferably 0.05 to 0.1 parts by weight of edetate, 0.02 to 0.05 parts by weight of sulfite and 0.001 to 0.02 parts by weight of dibutylhydroxytoluene, based on 100 parts by weight of the total weight of the pharmaceutical composition.

In addition, although there are no particular limitations on the ratio of butylhydroxyanisole in the pharmaceutical composition according to the present invention, it is generally 0.0001 to 0.003 parts by weight, preferably 0.001 to 0.003 parts by weight and more preferably 0.0020 to 0.0028 parts by weight of butylhydroxyanisole based on 100 parts by weight of the total weight of the pharmaceutical composition.

The pharmaceutical composition according to the present invention may blend additives such as, but are not limited to, vehicles, binders, disintegrating agents, suspending agents or emulsifiers, fragrances, sweeteners, fluidizing agents, colorants, pH adjusters, preservatives, solubilizers, solubilizing agents or ingredients of the base.
Specific examples of the vehicle may include, but are not limited to, D-mannitol, lactose (including anhydrous lactose), sucrose (including purified sucrose), sodium chloride, sodium bicarbonate, crystalline cellulose, light anhydrous silicic acid, anhydrous calcium phosphate, precipitated calcium carbonate and calcium silicate.
Specific examples of the binder may include, but are not limited to, dextrin, hydroxypropyl cellulose, hydroxypropyl methylcellulose, methylcellulose, polyvinyl alcohol, sodium carboxymethylcellulose, pullulan and powderd acacia.
Specific examples of the disintegrating agent may include, but are not limited to, low- substituted hydroxypropyl cellulose, carmellose, sodium carboxymethyl starch and crospovidone.
Specific examples of the suspending agent or emulsifier may include, but are not limited to, lecithin, sucrose fatty acid esters, polyglycerin fatty acid esters, polyoxyethylene hydrogenated castor oil, polysorbate and polyoxyethylene-polyoxypropylene copoylmer.
Specific examples of the fragrance may include, but are not limited to, menthol, peppermint oil, lemon oil and orange oil.
Specific examples of the sweetener may include, but are not limited to, saccharose, sorbitol, trehalose, maltitol, xylitol, aspartame, acesulfame potassium, sucrose, thaumatin and saccharin sodium.
Specific examples of the fluidizing agent may include, but are not limited to, hydrated silicon dioxide, light anhydrous silicic acid, heavy anhydrous silicic acid, crystalline cellulose, synthetic aluminum silicate, aluminum magnesium hydroxide, magnesium aluminometasilicate, stearic acid, calcium stearate, magnesium stearate, tricalcium phosphate and talc.
Specific examples of the colorant may include, but are not limited to, tar dyes such as food yellow no. 4, food yellow no. 5, food red no. 2, food red no. 102, food blue no. 1, food blue no. 2 (indigo carmine) or food yellow no. 4 aluminum lake, titanium oxide, yellow iron sesquioxide, iron sesquioxide, zinc oxide, talc, turmeric extract, caramel, liquid carotene, β-carotene, copper chlorophyll, sodium copper chlorophyllin, riboflavin, carbon black and medicinal carbon.
Specific examples of the pH adjuster may include, but are not limited to, hydrochloric acid, citric acid and salts thereof, tartaric acid and salts thereof, acetic acid and salts thereof, potassium hydroxide, calcium hydroxide, sodium hydroxide, magnesium hydroxide, sodium bicarbonate, sodium carbonate, lactic acid and salts thereof, phosphoric acid and salts thereof and malic acid.
Specific examples of the preservative may include, but are not limited to, benzoic acid and salts thereof, sorbic acid and salts thereof, dehydroacetic acid and salts thereof, methyl parahydroxybenzoate, ethyl parahydroxybenzoate, propyl parahydroxybenzoate and butyl parahydroxybenzoate.
Specific examples of the solubilizer may include, but are not limited to, ethanol, glycerin, propylene glycol, dilute hydrochloric acid, hydrogenated oils, purified water, physiological saline, water for injection, Macrogol 4000 and Polysorbate 80.
Specific examples of the solubilizing agent may include, but are not limited to, ethanol, hydrochloric acid, sodium hydroxide, glycine, glycerin, propylene glycol, cyclodextrin, liquid paraffin, hydrogenated castor oil, Macrogol 4000 and Polysorbate 80.
Specific examples of the ingredients of the base may include, but are not limited to, polymer substances such as acrylic polymers, cellulose polymers, polyisobutylene, rosin-based resins or rubber, waxes such as vaseline, synthetic polymer substances such as macrogols, hydrogenated oils and purified water.

The process of producing the pharmaceutical composition according to the present invention comprises a step of mixing donepezil or a pharmaceutically acceptable salt thereof, a naturally-occurring polymer, and at least one of edetate, sulfite, dibutylhydroxytoluene and butylhydroxyanisole. These constituents may be added separately or mixed together at one time, provided that they are ultimately mixed in the pharmaceutical composition.

Similarly, the method for stabilizing the pharmaceutical composition according to the present invention comprises a step of mixing donepezil or a pharmaceutically acceptable salt thereof, a naturally-occurring polymer, and at least one of edetate, sulfite, dibutylhydroxytoluene and butylhydroxyanisole. The donepezil or pharmaceutically acceptable salt thereof is stabilized, provided that these constituents are ultimately mixed in the pharmaceutical composition, regardless of whether these constituents are added separately or mixed together at one time.

In this manner, in the present invention, the edetate, sulfite, dibutylhydroxytoluene or butylhydroxyanisole functions as a stabilizer of the donepezil or pharmaceutically acceptable salt thereof in the pharmaceutical composition containing donepezil or pharmaceutically acceptable salt thereof and the naturally-occurring polymers.

The form of the pharmaceutical composition according to the present invention is not particularly limited, provided that it blends the naturally-occurring polymers and at least one of edetate, sulfite, dibutylhydroxytoluene and butylhydroxyanisole. Examples of such forms may include: oral preparations such as a syrup, suspension, dry syrup, liquid or jelly; suppositories; external preparations such as a lotion; and injectable preparations. Each of these forms can be produced according to conventionally known methods.

For example, a jelly can be produced by a process comprising a step of mixing donepezil or pharmaceutically acceptable salt thereof, a naturally-occurring polymer such as carrageenan, agar, pectin, xanthan gum, locust bean gum, gelatin or gua gum, at least one of edetate, sulfite, dibutylhydroxytoluene and butylhydroxyanisole, and water. In addition, the mixture can be gelled by a heating step or a step of mixing an acidic component or crosslinking agent such as calcium or potassium, as necessary. Sweeteners, fragrances, colorants, pH adjusters, preservatives, solubilizers or solubilizing agents and the like may also be suitably combined.

For example, a liquid preparation can be produced by a process comprising a step of mixing donepezil or pharmaceutically acceptable salt thereof, a naturally-occurring polymer such as carrageenan, agar, pectin, xanthan gum, locust bean gum, gelatin or gua gum, at least one of edetate, sulfite, dibutylhydroxytoluene and butylhydroxyanisole, and water. Emulsifiers, solubilizing agents, sweeteners, colorants, fragrances, pH adjusters, suspending agents, isotonic agents, buffers or preservatives and the like may also be suitably combined.

Furthermore, the pharmaceutical composition according to the present invention may be packed in one or more packaging forms selected from the group consisting of a oxygen scavenger-enclosed package, a gas-flushed package and a vacuum package.

### (Examples)

The following Examples provide a more detailed explanation of the present invention. These examples are shown for exemplary purposes only and should not be interpreted as limiting the scope of the present invention. In addition, additives which comply with the Japanese Pharmacopeia, Japanese Pharmaceutical Excipients, Japanese Standards of Food Additives and other official compendiums, or reagents are used for the additives contained in the pharmaceutical composition according to the present invention.

### (Examples 1 to 3)

Jelly preparations according to the present invention were prepared using the method described below with the formulae shown in Table 1.
9.8 kg of purified water were weighed into a 30 L stainless steel tank, and 22.0 g of calcium disodium edetate was dissolved therein with a disperser rotating at a speed of 1000 rpm. 440 g of pectin (LM Pectin) and 6.6 kg of powdered hydrogenated maltose starch syrup were then added and dispersed for 15 minutes. After heating to 85°C, 22.0 g of sodium benzoate was added. 94.6 g of citric acid dissolved in 700 g of purified water was then added to adjust the pH to 3.6. Subsequently, 6.6 g of donepezil hydrochloride (11.0 g in Example 1, 22.0 g in Example 3) was dissolved in 800 g of purified water and added. 22.0 g of calcium lactate was dissolved in 2200 g of purified water and added gradually and finally, 22.0 g of fragrance was added. After adjusting to a total weight of 22 kg with purified water, the mixture was cooled to 60°C while stirring and then, filled into a suitable container. After allowing it cool to room temperature, a composition according to the present invention was obtained.

**[Table 1]**

| Component | Manufacturer | Formula (%) | | |
|---|---|---|---|---|
| | | Example 1 | Example 2 | Example 3 |
| Donepezil hydrochloride | Eisai Co., Ltd. | 0.030 | 0.050 | 0.100 |
| Pectin | Sansho Co., Ltd. | 2.0 | 2.0 | 2.0 |
| Calcium lactate | Tomita Pharmaceutical Co., Ltd. | 0.10 | 0.10 | 0.10 |
| Powdered hydrogenated maltose starch syrup | Towa Chemical Industry Co., Ltd. | 30.00 | 30.00 | 30.00 |
| Citric acid | Satsuma Kako Co., Ltd. | q.s. | q.s. | q.s. |
| | | (pH 3.6) | (pH 3.6) | (pH 3.6) |
| Sodium benzoate | Fushimi Pharmaceutical Co., Ltd. | 0.10 | 0.10 | 0.10 |
| Calcium disodium edetate | Chelest Corp. | 0.10 | 0.10 | 0.10 |
| Fragrance | Takasago International Corp. | 0.10 | 0.10 | 0.10 |
| Purified water | Eisai Co., Ltd. | q.s. | q.s. | q.s. |

### (Example 4)

A jelly preparation according to the present invention was prepared using the method described below with the formula shown in Table 2.
300 g of purified water were weighed into a 2 L stainless steel beaker, and 1.0 g of calcium disodium edetate, 14 g of disodium hydrogen phosphate and 0.6 g of citric acid were dissolved therein with a stirrer rotating at a speed of 200 rpm. 3 g of kappa-carrageenan, 10 g of iota-carrageenan, 200 g of trehalose and 3.3 g of acesulfame potassium were then added and dispersed for 15 minutes. After heating to 90°C, 1.0 g of sodium benzoate was added. 0.667 g of donepezil hydrochloride was then dissolved in 30 g of purified water and added. Subsequently, 1.0 g of potassium chloride and 4.0 g of calcium lactate were dissolved in 300 g of purified water and added slowly and finally, 1.0 g of fragrance was added. After adjusting to a total weight of 1.0 kg with purified water, the mixture was cooled to 60°C while stirring and then, filled into a suitable container. After allowing it cool to room temperature, a composition according to the present invention was obtained.

**[Table 2]**

| Component | Manufacturer | Formula |
|---|---|---|
| | | (%) |
| Donepezil hydrochloride | Eisai Co., Ltd. | 0.0667 |
| Kappa-carrageenan | Marine Science Co., Ltd. | 0.300 |
| lota-carrageenan | Marine Science Co., Ltd. | 1.000 |
| Citric acid | Satsuma Kako Co., Ltd. | 0.060 |
| Potassium chloride | Tomita Pharmaceutical Co., Ltd. | 0.100 |
| Calcium lactate | Tomita Pharmaceutical Co., Ltd. | 0.400 |
| Disodium hydrogen phosphate (12 hydrate) | Wako Pure Chemical Industries, Ltd. | 1.400 |
| Sodium benzoate | Fushimi Pharmaceutical Co., Ltd. | 0.10 |
| Trehalose | Asahi Kasei Chemicals Corp. | 20.000 |
| Acesulfame potassium | Nutrinova | 0.33 |
| Fragrance | Takasago International Corp. | 0.100 |
| Calcium disodium edetate | Chelest Corp. | 0.100 |
| Purified water | Eisai Co., Ltd. | q.s. |

### (Examples 5 to 7)

Jelly preparations according to the present invention were prepared using the method described below with the formulae shown in Table 3.
4 kg of purified water was weighed into a 20 L stainless steel tank, and 10.0 g of calcium disodium edetate and 30 g of citric acid were dissolved therein with a disperser rotating at a speed of 1000 rpm. 100 g of agar and 3.0 kg of powdered hydrogenated maltose starch syrup were then added and dispersed for 15 minutes. After heating to 90°C, 10.0 g of sodium benzoate was added. 1.0 g of ethyl paraben were dissolved in 100 g of propylene glycol and added. Subsequently, 3.0 g of donepezil hydrochloride (5.0 g in Example 6, 10.0 g in Example 7) was dissolved in 800 g of purified water and added. 30 g of sodium citrate was dissolved in 570 g of purified water and added to adjust the pH to 5.0. Finally, 10.0 g of fragrance was added. After adjusting to a total weight of 10 kg with purified water, the mixture was cooled to 60°C while stirring and then, filled into a suitable container. After allowing it cool to room temperature, a composition according to the present invention was obtained.

**[Table 3]**

| Component | Manufacturer | Formula (%) | | |
|---|---|---|---|---|
| | | Example 5 | Example 6 | Example 7 |
| Donepezil hydrochloride | Eisai Co., Ltd. | 0.030 | 0.050 | 0.100 |
| Agar | Ina Food Industry Co., Ltd. | 1.0 | 1.0 | 1.0 |
| Powdered hydrogenated maltose starch syrup | Towa Chemical Industry Co., Ltd. | 30.00 | 30.00 | 30.00 |
| Citric acid | Satsuma Kako Co., Ltd. | 0.30 | 0.30 | 0.30 |
| Sodium citrate | Satsuma Kako Co., Ltd. | q.s. | q.s. | q.s. |
| | | (pH 5.0) | (pH 5.0) | (pH 5.0) |
| Sodium benzoate | Fushimi Pharmaceutical Co., Ltd. | 0.10 | 0.10 | 0.10 |
| Ethyl paraben | Ueno Fine Chemicals Industry, Ltd. | 0.01 | 0.01 | 0.01 |
| Propylene glycol | Adeka Corp. | 1.0 | 1.0 | 1.0 |
| Calcium disodium edetate | Chelest Corp. | 0.10 | 0.10 | 0.10 |
| Fragrance | Takasago International Corp. | 0.10 | 0.10 | 0.10 |
| Purified water | Eisai Co., Ltd. | q.s. | q.s. | q.s. |

### (Examples 8 to 10)

Jelly preparations according to the present invention were prepared using the method described below with the formulae shown in Table 4.
9.8 kg of purified water was weighed into a 30 L stainless steel tank, and 22.0 g of calcium disodium edetate was dissolved therein with a disperser rotating at a speed of 1000 rpm. 330 g of pectin (LM Pectin), 33 g of iota-carrageenan and 6.6 kg of powdered hydrogenated maltose starch syrup were then added and dispersed for 15 minutes. After heating to 85°C, 22.0 g of sodium benzoate and 72.6 g of acesulfame potassium were added. 2.64 g of ethyl paraben were dissolved in 660 g of propylene glycol and added. Subsequently, 94.6 g of citric acid were dissolved in 700 g of purified water and added. Furthermore, 6.6 g of donepezil hydrochloride (11.0 g in Example 9, 22.0 g in Example 10) was dissolved in 800 g of purified water and added. 22.0 g of calcium lactate was dissolved in 2200 g of purified water and added gradually and finally, 22.0 g of fragrance was added. After adjusting to a total weight of 22 kg with purified water, the mixture was cooled to 60°C while stirring and then, filled into a suitable container. After allowing it cool to room temperature, a composition according to the present invention was obtained.

**[Table 4]**

| Component | Manufacturer | Formula (%) | | |
|---|---|---|---|---|
| | | Example 8 | Example 9 | Example 10 |
| Donepezil hydrochloride | Eisai Co., Ltd. | 0.030 | 0.050 | 0.100 |
| Pectin | Sansho Co., Ltd. | 1.5 | 1.5 | 1.5 |
| lota-carrageenan | Marine Science Co., Ltd. | 0.15 | 0.15 | 0.15 |
| Powdered hydrogenated maltose starch syrup | Towa Chemical Industry Co., Ltd. | 30.00 | 30.00 | 30.00 |
| Acesulfame potassium | Nutrinova | 0.33 | 0.33 | 0.33 |
| Citric acid | Satsuma Kako Co., Ltd. | 0.43 | 0.43 | 0.43 |
| Calcium lactate | Tomita Pharmaceutical Co., Ltd. | 0.1 | 0.1 | 0.1 |
| Sodium citrate | Satsuma Kako Co., Ltd. | q.s. | q.s. | q.s. |
| Sodium benzoate | Fushimi Pharmaceutical Co., Ltd. | 0.10 | 0.10 | 0.10 |
| Ethyl paraben | Ueno Fine Chemicals Industry, Ltd. | 0.012 | 0.012 | 0.012 |
| Propylene glycol | Adeka Corp. | 3.0 | 3.0 | 3.0 |
| Calcium disodium edetate | Chelest Corp. | 0.10 | 0.10 | 0.10 |
| Fragrance | Takasago International Corp. | 0.10 | 0.10 | 0.10 |
| Purified water | Eisai Co., Ltd. | q.s. | q.s. | q.s. |

### (Examples 11 and 12)

Jelly preparations according to the present invention were prepared using the method described below with the formulae shown in Table 5.
9 kg of purified water was weighed into a 30 L stainless steel tank, and 4.4 g of dibutylhydroxytoluene (0.66 g of butylhydroxyanisole in Example 12) was dissolved therein with a disperser rotating at a speed of 1000 rpm. 374 g of pectin (LM Pectin), 22 g of iota-carrageenan and 6.6 kg of powdered hydrogenated maltose starch syrup were then added and dispersed for 15 minutes. After heating to 85°C, 22.0 g of sodium benzoate and 72.6 g of acesulfame potassium was added. 2.64 g of ethyl paraben was dissolved in 660 g of propylene glycol and added, after which 4.4 g of donepezil hydrochloride was dissolved in 800 g of purified water and added. 37.4 g of calcium lactate was dissolved in 2200 g of purified water and added gradually followed by the addition of 22.0 g of fragrance. Finally, 150 g of citric acid was dissolved in 1500 g of purified water and added to adjust the pH to 3.6. After adjusting to a total weight of 22 kg with purified water, the mixture was cooled to 60°C while stirring and then, filled into a suitable container. After allowing it cool to room temperature, a composition according to the present invention was obtained.

**[Table 5]**

| Component | Manufacturer | Formula (%) | |
|---|---|---|---|
| | | Example 11 | Example 12 |
| Donepezil hydrochloride | Eisai Co., Ltd. | 0.020 | 0.020 |
| Pectin | Sansho Co., Ltd. | 1.700 | 1.700 |
| lota-carrageenan | Marine Science Co., Ltd. | 0.10 | 0.10 |
| Citric acid | Satsuma Kako Co., Ltd. | q.s. | q.s. |
| | | (pH 3.6) | (pH 3.6) |
| Calcium lactate | Tomita Pharmaceutical Co., Ltd. | 0.17 | 0.17 |
| Powdered hydrogenated maltose starch syrup | Towa Chemical Industry Co., Ltd. | 30.0 | 30.0 |
| Sodium benzoate | Fushimi Pharmaceutical Co., Ltd. | 0.10 | 0.10 |
| Ethyl paraben | Ueno Fine Chemicals Industry, Ltd. | 0.012 | 0.012 |
| Propylene glycol | Adeka Corp. | 3.0 | 3.0 |
| Acesulfame potassium | Nutrinova | 0.33 | 0.33 |
| Fragrance | Takasago International Corp. | 0.10 | 0.10 |
| Dibutylhydroxytoluene | Wako Pure Chemical Industries, Ltd. | 0.020 | - |
| Butylhydroxyanisole | Wako Pure Chemical Industries, Ltd. | - | 0.003 |
| Purified water | Eisai Co., Ltd. | q.s. | q.s. |

### (Comparative Example 1)

Comparative Example 1 which did not contain a stabilizer was prepared using the method described below.
9 kg of purified water was weighed into a 30 L stainless steel tank, and 374 g of pectin (LM Pectin), 22 g of iota-carrageenan and 6.6 kg of powdered hydrogenated maltose starch syrup were added and dispersed for 15 minutes. After heating to 85°C, 22.0 g of sodium benzoate and 72.6 g of acesulfame potassium were added. 2.64 g of ethyl paraben was dissolved in 660 g of propylene glycol and added, after which 4.4 g of donepezil hydrochloride was dissolved in 800 g of purified water and added. 37.4 g of calcium lactate was dissolved in 2200 g of purified water and added gradually followed by the addition of 22.0 g of fragrance. Finally, 150 g of citric acid was dissolved in 1500 g of purified water and added to adjust the pH to 3.6. After adjusting to a total weight of 22 kg with purified water, the mixture was cooled to 60°C while stirring and then, filled into a suitable container. After allowing it cool to room temperature, Comparative Example 1 was obtained.

### (Comparative Examples 2 to 6 and Examples 13 to 17)

Comparative Examples 2 to 6 and Examples 13 to 17 were prepared using the method described below with the formula of Table 6.
9 kg of purified water was weighed into a 30 L stainless steel tank, and 4.4 g of propyl gallate (4.4 g of ascorbyl stearate ester in Comparative Example 3, 4.4 g of sodium ascorbate in Comparative Example 4, 4.4 g of ascorbic acid in Comparative Example 5, 4.4 g of cysteine hydrochloride in Comparative Example 6, 4.4 g of sodium sulfite in Example 13, 4.4 g of sodium pyrosulfite in Example 14, 4.4 g of sodium edetate in Example 15, 4.4 g of dibutylhydroxytoluene in Example 16 or 0.66 g of butylhydroxyanisole in Example 17) as a stabilizer was dissolved therein with a disperser rotating at a speed of 1000 rpm. Sebsequently, 374 g of pectin (LM Pectin), 22 g of iota-carrageenan and 6.6 kg of powdered hydrogenated maltose starch syrup were added and dispersed for 15 minutes. After heating to 85°C, 22.0 g of sodium benzoate and 72.6 g of acesulfame potassium were added. 2.64 g of ethyl paraben was dissolved in 660 g of propylene glycol and added, after which 4.4 g of donepezil hydrochloride was dissolved in 800 g of purified water and added. 37.4 g of calcium lactate was dissolved in 2200 g of purified water and added gradually followed by the addition of 22.0 g of fragrance. Finally, 150 g of citric acid was dissolved in 1500 g of purified water and added to adjust the pH to 3.6. After adjusting to a total weight of 22 kg with purified water, the mixture was cooled to 60°C while stirring and then, filled into a suitable container. After allowing it cool to room temperature, a composition of the present invention was obtained.

**[Table 6]**

| Component | Manufacturer | Formula (%) |
|---|---|---|
| Donepezil hydrochloride | Eisai Co., Ltd. | 0.020 |
| Stabilizer | - | 0.02% |
| | | (0.003% for BHA) |
| Pectin | Sansho Co., Ltd. | 1.7 |
| lota-carrageenan | Marine Science Co., Ltd. | 0.10 |
| Calcium lactate | Tomita Pharmaceutical Co., Ltd. | 0.17 |
| Powdered hydrogenated maltose starch syrup | Towa Chemical Industry Co., Ltd. | 30.0 |
| Citric acid | Satsuma Kako Co., Ltd. | q.s. |
| Sodium benzoate | Fushimi Pharmaceutical Co., Ltd. | 0.10 |
| Ethyl paraben | Ueno Fine Chemicals Industry, Ltd. | 0.012 |
| Propylene glycol | Adeka Corp. | 3.0 |
| Acesulfame potassium | Nutrinova | 0.33 |
| Fragrance | Takasago International Corp. | 0.10 |
| Purified water | Eisai Co., Ltd. | q.s. |

| | | |
|---|---|---|
| Stabilizer: propyl gallate (Comparative Example 2), ascorbyl stearate ester (Comparative Example 3), sodium ascorbate (Comparative Example 4), ascorbic acid (Comparative Example 5), cysteine hydrochloride (Comparative Example 6), sodium sulfite (Example 13), sodium pyrosulfite (Example 14), sodium edetate (Example 15), dibutylhydroxytoluene (Example 16), or butylhydroxyanisole (also referred to as "BHA") (Example 17). | | |

### (Examples 18 to 20)

Jelly preparations according to the present invention were prepared using the method described below with the formulae shown in Table 7.
9 kg of purified water was weighed into a 30 L stainless steel tank, and 6.6 g of sodium edetate (0.44 g of sodium edetate in Example 19, 14.52 g of calcium disodium edetate in Example 20) was dissolved therein with a disperser rotating at a speed of 1000 rpm. Subsequently, 374 g of pectin (LM Pectin), 22 g of iota-carrageenan and 6.6 kg of powdered hydrogenated maltose starch syrup were added and dispersed for 15 minutes. After heating to 85°C, 22.0 g of sodium benzoate and 72.6 g of acesulfame potassium were added. 2.64 g of ethyl paraben was dissolved in 660 g of propylene glycol and added, after which 4.4 g of donepezil hydrochloride was dissolved in 800 g of purified water and added. 37.4 g of calcium lactate was dissolved in 2200 g of purified water and added gradually followed by the addition of 22.0 g of fragrance. Finally, 150 g of citric acid was dissolved in 1500 g of purified water and added to adjust the pH to 3.6. After adjusting to a total weight of 22 kg with purified water, the mixture was cooled to 60°C while stirring and then, filled into a suitable container. After allowing it cool to room temperature, a composition of the present invention was obtained.

**[Table 7]**

| Component | Manufacturer | Formula (%) | | |
|---|---|---|---|---|
| | | Example 18 | Example 19 | Example 20 |
| Donepezil hydrochloride | Eisai Co., Ltd. | 0.020 | 0.020 | 0.020 |
| Sodium edetate | Chelest Corp. | 0.030 | 0.002 | - |
| Calcium disodium edetate | Wako Pure Chemical Industries, Ltd. | - | - | 0.066 |
| Pectin | Sansho Co., Ltd. | 1.7 | 1.7 | 1.7 |
| lota-carrageenan | Marine Science Co., Ltd. | 0.10 | 0.10 | 0.10 |
| Calcium lactate | Tomita Pharmaceutical Co., Ltd. | 0.17 | 0.17 | 0.17 |
| Powdered hydrogenated maltose starch syrup | Towa Chemical Industry Co., Ltd. | 30.0 | 30.0 | 30.0 |
| Citric acid | Satsuma Kako Co., Ltd. | q.s. | q.s. | q.s. |
| Sodium benzoate | Fushimi Pharmaceutical Co., Ltd. | 0.10 | 0.10 | 0.10 |
| Ethyl paraben | Midori Kagaku Co., Ltd. | 0.012 | 0.012 | 0.012 |
| Propylene glycol | Adeka Corp. | 3.0 | 3.0 | 3.0 |
| Acesulfame potassium | Nutrinova | 0.33 | 0.33 | 0.33 |
| Fragrance | Takasago International Corp. | 0.10 | 0.10 | 0.10 |
| Purified water | Eisai Co., Ltd. | q.s. | q.s. | q.s. |

### (Experimental Example 1) Storage Stability Test

According to the present invention, the stability of donepezil or pharmaceutically acceptable salt thereof with respect to heat or change over time is remarkably enhanced. This effect of the present invention is verified by showing the results for examples and comparative examples.

### (Storage Stability Test 1)

Each of the compositions of Comparative Examples 1 to 6 and Examples 13 to 17 of the present invention was sealed in a storage container which is a polypropylene cup covered with aluminum. The compositions were stored for 1 month under storage conditions of a temperature of 40°C and relative humidity of 75% after which stability was tested by measuring the content of related substances.

### (Method of Measuring Content)

The content of related substances was measured with the high-performance liquid chromatography (HPLC) operating conditions shown in Table 8. The content of related substances was calculated from the ratio of the peak area for donepezil to the peak area for the related substance.

**[Table 8] HPLC Operating Conditions**

| | |
|---|---|
| Detector | UV absorption detector (measuring wavelength: 271 nm) or PDA |
| Column | Stainless steel tube having an inner diameter of 4.6 mm and length of 150 mm packed with 5 µm octadecylsilylated silica gel for liquid chromatography |
| Column temperature | Constant temperature of about 40°C |
| Flow rate | Approx. 1.3 mL/min |
| Mobile phase | Mixture of water, acetonitrile and perchloric acid (1300:700:1) containing 10 mmol/L of sodium decane sulfonate |
| Diluent | Mixture of methanol and 0.1 mol/L hydrochloric acid (1:1) |
| Injection volume | 50 µL |
| Analysis time | 30 minutes |

Results of the detection of the amounts of related substances are shown in Table 9. In the present invention, compositions which contain sodium edetate, dibutylhydroxytoluene or butylhydroxyanisole demonstrated an increase in stability of donepezil or pharmaceutically acceptable salt thereof with hardly any related substances detected, in comparison with the composition of Comparative Example 1 which does not contain a stabilizer. The formation of related substances that increase due to the coexistence of a naturally-occurring polymer and donepezil or pharmaceutically acceptable salt thereof was confirmed to be suppressed to an extremely small amount (0.09% or less) or to an amount below the detection limit, when sodium edetate, sodium sulfite, sodium pyrosulfite, dibutylhydroxytoluene or butylhydroxyanisole was added. In contrast, it was confirmed that, in comparison with compositions of the present invention, the formation of related substances in the pharmaceutical compositions was not suppressed when one of ordinary stabilizers - propyl gallate, ascorbyl stearate ester and sodium ascorbate was used.
It was also confirmed that the related substances that form in the case of coexistence of the naturally-occurring polymer and donepezil or pharmaceutically acceptable salt thereof had different retention times under the same HPLC conditions from those of related substances formed in the case of coexistence of PVP as a synthetic polymer and donepezil or pharmaceutically acceptable salt thereof. This means that the related substances formed in the case of coexistence of the naturally-occurring polymers and donepezil or pharmaceutically acceptable salt thereof is different from the related substances formed in the case of coexistence of PVP as a synthetic polymer and donepezil or pharmaceutically acceptable salt thereof.

**[Table 9]**

| Stabilizer | Related substances |
|---|---|
| | (%) |
| None | 0.30 |
| Propyl gallate | 0.69 |
| Ascorbyl stearate ester | 0.41 |
| Sodium ascorbate | 0.36 |
| Ascorbic acid | 0.26 |
| Cysteine hydrochloride | 0.22 |
| Sodium sulfite | 0.09 |
| Sodium pyrosulfite | 0.03 |
| Sodium edetate | - |
| Dibutylhydroxytoluene | - |
| Butylhydroxyanisole | - |

### (Storage Stability Test 2)

Each of the compositions of Examples 15, 18 and 19 and Comparative Example 1 of the present invention was sealed in a storage container which is a polypropylene cup covered with aluminum. The compositions were stored for 1, 3 or 6 months under storage conditions of a temperature of 40°C and relative humidity of 75% after which stability was tested by measuring the content of related substances.

Results of the detection of amounts of related substances using the previously described method of measuring content are shown in Table 10. In the present invention, hardly any related substances were detected in the compositions to which sodium edetate was added, regardless of the amount of the sodium edetate. Compared with the composition of Comparative Example 1 that did not contain a stabilizer, the stability of donepezil or pharmaceutically acceptable salt thereof was increased.
The formation of related substances that increase due to the coexistence of a naturally-occurring polymer and donepezil or pharmaceutically acceptable salt thereof was confirmed to be suppressed to an amount below the detection limit, with addition of sodium edetate.

**[Table 10]**

| Stabilizer (%) | Related Substances (%) | | |
|---|---|---|---|
| | After 1 month | After 3 months | After 6 months |
| None (Comparative Example 1) | 0.30 | 0.76 | 1.75 |
| Sodium edetate (0.020%, Example 15) | - | - | - |
| Sodium edetate (0.030%, Example 18) | - | - | - |
| Sodium edetate (0.002%, Example 19) | - | - | - |

### (Storage Stability Test 3)

Each of the compositions of Example 20 and Comparative Example 1 of the present invention was sealed in a storage container which is a polypropylene cup covered with aluminum. The compositions were stored for 1, 3 or 6 months under storage conditions of a temperature of 40°C and relative humidity of 75% after which stability was tested by measuring the content of related substances.

Results of the detection of the amount of related substances using the previously described method of measuring content are shown in Table 11. In the present invention, hardly any related substances were detected in the composition of Example 20 to which calcium disodium edetate was added, and the stability of donepezil or pharmaceutically acceptable salt thereof was increased, as compared to the composition of Comparative Example 1 that did not contain a stabilizer.
The formation of related substances that increase due to the coexistence of a naturally-occurring polymer and donepezil or pharmaceutically acceptable salt thereof is suppressed to an amount below the detection limit, with addition of calcium disodium edetate.

**[Table 11]**

| Stabilizer (%) | Related Substances (%) | | |
|---|---|---|---|
| | After 1 month | After 3 months | After 6 months |
| None (Comparative Example 1) | 0.30 | 0.76 | 1.75 |
| Calcium disodium edetate (0.066%, Example 20) | - | - | - |

### (Storage Stability Test 4)

Each of the compositions of Examples 8 to 10 of the present invention was sealed in a storage container which is a polypropylene cup covered with aluminum. The compositions were stored for 1, 3 or 6 months under storage conditions of a temperature of 40°C and relative humidity of 75% after which stability was tested by measuring the content of related substances.

Results of the detection of the amounts of related substances using the method described in the method of measuring content are shown in Table 12. It was confirmed that, according to the present invention, in the compositions of Examples 8 to 10 to which calcium disodium edetate was added, the formation of related substances after the storage for 1, 3 or 6 months was suppressed to an amount below the detection limit, even in the case where the amount of donepezil blended therein is increased, and the stability of donepezil or pharmaceutically acceptable salt thereof was increased.
The formation of related substances that increase due to the coexistence of a naturally-occurring polymer and donepezil or pharmaceutically acceptable salt thereof was confirmed to be suppressed to an amount below the detection limit with the addition of calcium disodium edetate.

**[Table 12]**

| Stabilizer (%) | Related Substances (%) | | |
|---|---|---|---|
| | After 1 month | After 3 months | After 6 months |
| Calcium disodium edetate (0.1 %, Example 8) | - | - | - |
| Calcium disodium edetate (0.1% Example 9) | - | - | - |
| Calcium disodium edetate (0.1%, Example 10) | - | - | - |

### (Example of Oxygen scavenger-enclosed Packaging Form)

A polypropylene film was molded into the shape of a cup and 10 g of dissolved composition obtained by dissolving each of the compositions of Examples 8 to 10 and thereafter cooling to 60°C while stirring was filled therein, using a container molding and filling machine (CKD Corp., CFF-200). The cups were then immediately heat-sealed with an aluminum film and cut out.
The filled cup packages were placed in a sterilizer (Hisaka Works, Ltd.) and sterilized for 30 minutes at 85°C. Subsequently, the packages were cooled to room temperature to solidify into jelly. The cup packages were then placed in an aluminum pouches together with a oxygen scavenger (Ageless Z-20PT, Mitsubishi Gas Chemical Co., Inc.) and sealed to obtain pharmaceutical compositions in the oxygen scavenger-enclosed packaging forms.

### (Example 21)

A liquid preparation according to the present invention was prepared using the method described below with the formula shown in Table 13.
120 kg of purified water was placed in a 400 L stainless steel tank and 378.5 g of calcium disodium edetate and 757 g of citric acid were added. After stirring for 1 minute with a propeller rotating at 200 rpm, 378.5 g of donepezil hydrochloride was added and stirred for 10 minutes at 400 rpm. Subsequently, 1892.5 g of iota-carrageenan was added, and while stirring at 200 rpm, 135.1 kg of 70% D-sorbitol solution was further added. In a separate stainless steel tank, 22.71 kg of propylene glycol was placed and 378.5 g of methyl paraben was added. After stirring for 10 minutes with a compact stirrer at 900 rpm, 189.3 g of fragrance was added and additionally stirred for 1 minute. 100 kg of purified water was further added to this solution and stirred for 1 minute at 200 rpm. In a separate stainless steel tank, 9 kg of purified water was placed, 378.5 g of sodium benzoate was added and stirred for 10 minutes with a compact stirrer at 900 rpm. This solution was placed in the 400 L stainless steel tank and stirred for 3 minutes at 200 rpm. After adjusting the pH to 4.0 with 10% sodium citrate aqueous solution, the total weight was adjusted to 416.9 kg with purified water and filtered to obtain a composition according to the present invention.

**[Table 13]**

| Component | Manufacturer | Formula |
|---|---|---|
| | | (mg/5 ml) |
| Donepezil hydrochloride | Eisai Co., Ltd. | 5 |
| 70% D-sorbitol | Towa Chemical Industry Co., Ltd. | 1785 |
| Iota-carrageenan | Marine Science Co., Ltd. | 25 |
| Citric acid | Satsuma Kako Co., Ltd. | 10 |
| Sodium citrate | Satsuma Kako Co., Ltd. | q.s. (pH 4.0) |
| Sodium benzoate | Fushimi Pharmaceutical Co., Ltd. | 5 |
| Methyl paraben | Ueno Fine Chemicals Industry, Ltd. | 5 |
| Propylene glycol | Adeka Corp. | 300 |
| Disodium calcium edetate | Chelest Corp. | 5 |
| Fragrance | Takasago International Corp. | 15 |
| Purified water | Eisai Co., Ltd. | q.s. |
| Total (ml) | | 5.00 |

### INDUSTRIAL APPLICABILITY

According to the present invention, a pharmaceutical composition containing donepezil or pharmaceutically acceptable salt thereof and a naturally-occurring polymer that improves patient compliance, prevents accidental swallowing in patients with swallowing disorders and reduces management burden during the course of formulation is provided.

## Claims

1. A pharmaceutical composition comprising donepezil or a pharmaceutically acceptable salt thereof and a naturally-occurring polymer,
wherein the pharmaceutical composition further comprises at least one of edetate, sulfite, dibutylhydroxytoluene and butylhydroxyanisole.

2. The pharmaceutical composition according to Claim 1, wherein the edetate is sodium edetate or calcium disodium edetate.

3. The pharmaceutical composition according to Claim 1, wherein the sulfite is sodium sulfite, sodium bisulfite, potassium sulfite, sodium pyrosulfite or potassium pyrosulfite.

4. The pharmaceutical composition according to any one of Claims 1 to 3, wherein the naturally-occurring polymer is selected from the group consisting of pectin, carrageenan, agar, gelatin, gua gum, psyllium seed gum, xanthan gum, locust bean gum, starch, sodium alginate, tara gum, tamarind gum, glucomannan, gellan gum, curdlan, pullulan, gum arabic and mixtures thereof.

5. The pharmaceutical composition according to any one of Claims 1 to 4, wherein an amount of the edetate, sulfite or dibutylhydroxytoluene is 0.0001 to 0.2 parts by weight based on 100 parts by weight of the total weight of the pharmaceutical composition.

6. The pharmaceutical composition according to any one of Claims 1 to 4, wherein an amount of the butylhydroxyanisole is 0.0001 to 0.003 parts by weight based on 100 parts by weight of the total weight of the pharmaceutical composition.

7. The pharmaceutical composition according to any one of Claims 1 to 6, wherein the donepezil or pharmaceutically acceptable salt thereof is donepezil hydrochloride.

8. A process of producing a pharmaceutical composition comprising donepezil or a pharmaceutically acceptable salt thereof and a naturally-occurring polymer, comprising:
mixing at least one of edetate, sulfite, dibutylhydroxytoluene and butylhydroxyanisole into said pharmaceutical composition.

9. A method for stabilizing a pharmaceutical composition comprising donepezil or a pharmaceutically acceptable salt thereof and a naturally-occurring polymer, comprising:
adding at least one of edetate, sulfite, dibutylhydroxytoluene and butylhydroxyanisole to said pharmaceutical composition.

10. A stabilizer for a pharmaceutical composition comprising donepezil or a pharmaceutically acceptable salt thereof and a naturally-occurring polymer,
wherein the stabilizer comprises at least one of edetate, sulfite, dibutylhydroxytoluene and butylhydroxyanisole.
